# EUROPÄISCHE PATENTSCHRIFT

(11) **EP 3 671 196 B1**
(45) Veröffentlichungstag und Bekanntmachung des Hinweises auf die Patenterteilung: **16.11.2022**
(21) Anmeldenummer: 18214286.9
(22) Anmeldetag: 20.12.2018
(51) Int. Cl.: G01N 27/22, G01N 33/44

(54) **SYSTEM ZUR ERMITTLUNG EINES ALTERUNGSZUSTANDS EINES ELASTOMERPRODUKTS**
SYSTEM FOR DETERMINING AN AGING STATE OF AN ELASTOMERIC PRODUCT
SYSTÈME DE DÉTERMINATION DE L'ÉTAT DE VIEILLISSEMENT D'UN PRODUIT ÉLASTOMÈRIQUE

(43) Veröffentlichungstag der Anmeldung: 24.06.2020
(73) Patentinhaber: ContiTech AG, 30165 Hannover (DE)
(72) Erfinder: Bejinaru, Razvan, 710028 Botosani (RO); Iftode, Cora, 300243 Timisoara (RO)
(74) Vertreter: Continental Corporation

(56) Entgegenhaltungen:
- EP-A2- 2 375 099
- DE-A1-102014 116 497
- DE-A1-102014 213 969
- KANWAR KELASH ET AL: "Embedded UHF RFID tag design process for rubber transmission belt using 3D model", 2014 IEEE RFID TECHNOLOGY AND APPLICATIONS CONFERENCE (RFID-TA), IEEE, 8. September 2014 (2014-09-08), Seiten 97-102, XP032668639, DOI: 10.1109/RFID-TA.2014.6934208 [gefunden am 2014-10-22]

## Beschreibung

Die Erfindung betrifft ein System zur Ermittlung eines Alterungszustands eines Elastomerprodukts.

Elastomerprodukte sind grundsätzlich aus dem Stand der Technik bekannt. Hierbei kann es sich beispielsweise um ein Gummi-Riemen, ein Gummi-Förderband oder ein GummiLager handeln. Die Aufzählung ist nicht einschränkend zu verstehen. Grundsätzlich ist eine Vielzahl von unterschiedlichen Elastomerprodukten bekannt. Ein Elastomerprodukt weist ein erstes Elastomermaterial auf. Das erste Elastomermaterial kann von einem Gummi-Material gebildet sein.

Elastomerprodukte unterliegen einer Alterung, die durch das Alter des Elastomerprodukts als solches und/oder durch eine mechanische Belastung des Elastomerprodukts bestimmt sein kann. Durch ein zunehmendes Alter kann das Elastomerprodukt spröder werden und/oder an Zugfestigkeit verlieren. Für die praktische Verwendung eines Elastomerprodukts ist der Alterungszustand des Elastomerprodukts deshalb besonders interessant. Ist das Elastomerprodukt beispielsweise als ein Riemen ausgebildet, so kann der Alterungszustand des Riemens darüber Aufschluss ergeben, ob der Riemen der praktischen, mechanischen Anforderung Stand hält oder ob das Elastomerprodukt einen Alterungszustand hat, der nicht mehr den praktischen, mechanischen Anforderungen genügt.

Aus der DE 10 2014 213 969 A1 ist ein Verfahren zur Ermittlung des Alterungszustands eines Elastomerprodukts bekannt. Dabei wird ein Sensor in das Elastomermaterial des Elastomerprodukts eingebracht, um mittels des Sensors auf die Permittivität des Elastomermaterials des Elastomerprodukts zu schließen. Der Sensor wird mittels einer elektromagnetischen Welle angeregt, so dass der Sensor mit einer entsprechenden verstimmten elektromagnetischen Welle antwortet, die dazu verwendet wird, um auf den Alterungszustand des Elastomerprodukts zu schließen. In der Praxis wurde festgestellt, dass zur Verwendung des zuvor erläutertet Verfahrens eine sehr starke elektromagnetische Welle verwendet werden muss, damit die elektromagnetische Welle auch tatsächlich bis zu dem Sensor in dem Elastomerprodukt vordringt. Wird hingegen eine nicht sehr starke elektromagnetische Welle verwendet, was in der Praxis oftmals notwendig ist, so können äußere Störgrößen die Messung und damit auch das Ermittlungsergebnis stark beeinflussen. Dies ist jedoch zu vermeiden.

Der Erfindung liegt deshalb die Aufgabe zugrunde, ein System bereitzustellen, das eine störgrößenrobuste Ermittlung eines Alterungszustands eines Elastomerprodukts erlaubt. Gelöst wird die Aufgabe durch ein System mit den Merkmalen des Anspruchs 1. vorgesehen ist also ein System zur Ermittlung eines Alterungszustands eines Elastomerprodukts. Das System weist ein Elastomerprodukt mit einem ersten Elastomermaterial, einen Transceiver und eine Auswerteeinheit auf, die mit dem Transceiver gekoppelt ist. Das Elastomerprodukt weist einen Elastomerresonator und einen Metallresonator auf. Der Elastomerresonator ist in das erste Elastomermaterial des Elastomerprodukts eingebettet. Der Elastomerresonator ist von einem Streifen aus einem zweiten, elektrisch leitfähigen Elastomermaterial ausgebildet. Der Metallresonator ist an einer Außenseite des Elastomerprodukts angeordnet, so dass der Metallresonator und der Elastomerresonator gegenüberliegend und kontaktfrei zueinander angeordnet sind. Der Metallresonator, der Elastomerresonator und ein Teil des ersten Elastomermaterials, der zwischen dem Metallresonator und dem Elastomerresonator angeordnet sind, bilden einen Messresonator, dessen Reflektionskoeffizient von der Permittivität des ersten Elastomermaterials und von der elektrischen Leitfähigkeit des Elastomerresonators abhängt. Der Transceiver ist zur Erzeugung eines elektromagnetischen Wechselfelds ausgebildet. Der Messresonator ist ausgebildet, in das Wechselfeld eingekoppelt zu werden und das Wechselfeld derart zu verändern, so dass das Wechselfeld eine durch den Reflektionskoeffizienten des Messresonators geprägte Resonanzfrequenz aufweist. Der Transceiver ist zur Erfassung des Wechselfelds und zur Erzeugung eines Messsignals ausgebildet, dass das Wechselfeld und/oder die Resonanzfrequenz des Wechselfelds repräsentiert. Die Auswerteeinheit ist zur Ermittlung eines Alterungszustands des Elastomerprodukts basierend auf dem Messsignal ausgebildet.

Der Metallresonator ist vorzugsweise ein aus Metall gebildeter und/oder bestehender Resonator. Der Metallresonator kann auch als Metall-Antenne bezeichnet und/oder ausgebildet sein. Der Metallresonator kann ausgebildet sein, bei einer breitbandigen Anregung mit einer vorbestimmten Resonanzfrequenz zu schwingen. Der Metallresonator ist ein Teil des Messresonators. Die Resonanzfrequenz des Messresonators kann deshalb auf der Resonanzfrequenz des Metallresonators basieren und außerdem von den physikalischen Eigenschaften der direkt oder indirekt an den Metallresonator grenzenden Materialien ab, was durch die Permittivität des ersten Elastomermaterials und die Leitfähigkeit des Elastomerresonators bestimmt sein kann. Die Resonanzfrequenz des Messresonators kann deshalb von der Permittivität des ersten Elastomermaterials und von der elektrischen Leitfähigkeit des Elastomerresonators abhängen. Dieser Effekt wird genutzt, um besonders robust gegenüber äußeren Störgrößen auf den Alterungszustand des Elastomerprodukts zu schließen.

Das Elastomerprodukt weist das erste Elastomermaterial auf, in das der Elastomerresonator eingebettet ist. Der Elastomerresonator ist ein Resonator, der von einem Streifen aus einem zweiten, elektrisch leitfähigen Elastomermaterial gebildet ist. Der Resonator wird deshalb als Elastomerresonator bezeichnet. Vorzugsweise weist der zweite, elektrisch leifähige Elastomerresonator einen spezifischen Widerstand von weniger als 10.000 Ω^{∗}m, weniger als 1.000 Ω^{∗}m oder weniger als 100 Ω^{∗}m auf. Vorzugsweise besteht der Elastomerresonator ausschließlich aus dem zweiten, elektrisch leitfähigen Elastomermaterial. Das zweite, elektrisch leitfähige Elastomermaterial kann auf einem Elastomermaterial basieren, in das elektrisch leitfähige Partikel und/oder elektrisch leitfähige Füllstoffe eingebettet und/oder eingemischt sind. Die elektrisch leitfähigen Partikel bzw. Füllstoffe können beispielsweise aus der Gruppe von Rußpartikeln, Kohlenstoffpartikeln, Kohlenstoffnanoröhrchen, Graphenpartikeln, Graphitpartikel und/oder Metallpartikeln sein. Die elektrische Leitfähigkeit des zweiten, elektrisch leitfähigen Elastomermaterials kann jedoch auch durch einen anderen Füllstoff und/oder eine ionische Flüssigkeit, die in das entsprechende Elastomermaterial eingemischt und/oder eingebettet ist, gebildet sein.

Der Elastomerresonator ist in das erste Elastomermaterial des Elastomerprodukts eingebettet. Somit kann der Elastomerresonator vollständig von dem ersten Elastomermaterial umgeben sein. Besonders bevorzugt besteht zwischen dem Elastomerresonator und dem den Elastomerresonator umgebenden Teil des ersten Elastomermaterials ein vollständiger und/oder umfänglich umschlossener Kontakt. Der Elastomerresonator ist zu der Außenseite des Elastomerprodukts, an der der Metallresonator angeordnet ist, beabstandet. Der Abstand zwischen dem Elastomerresonator und dem Metallresonator kann vorbestimmt sein. Besonders bevorzugt sind der Elastomerresonator und der Metallresonator zumindest im Wesentlichen parallel zueinander angeordnet. Aufgrund des Abstands zwischen dem Metallresonator und dem Elastomerresonator ist dazwischen ein Teil des ersten Elastomermaterials des Elastomerprodukts angeordnet. Insbesondere wird der Bereich zwischen dem Metallresonator und dem Elastomerresonator vollständig von dem genannten Teil des ersten Elastomermaterials ausgefüllt.

In der Praxis wurde festgestellt, dass der Elastomerresonator und der Teil des Elastomermaterials, der zwischen dem Metallresonator und dem Elastomerresonator angeordnet ist, analog oder ähnlich wie ein Belastungswiderstand und/oder ein Belastungsbauteil wirken können, das mit dem Metallresonator direkt oder indirekt gekoppelt ist. Dies ist jedoch nicht zwingend der Fall. Der Metallresonator, der Elastomerresonator und der Teil des ersten Elastomermaterials, der zwischen dem Metallresonator und dem Elastomerresonator angeordnet ist, bilden zusammen den Messresonator. Hierbei handelt es sich ebenfalls um einen Resonator, der als Messresonator bezeichnet ist. Der Reflektionskoeffizient des Messresonators hängt von der Permittivität des ersten Elastomermaterials und von der elektrischen Leitfähigkeit des Elastomerresonators ab. Entsprechendes gilt für die Resonanzfrequenz des Messresonators. Die folgenden Erläuterungen zum Reflektionskoeffizienten des Messresonators können deshalb in analoger Weise für die Resonanzfrequenz des Messresonators gelten. Insbesondere kann beispielsweise die Resonanzfrequenz des Messresonators von der Permittivität des ersten Elastomermaterials und von der elektrischen Leitfähigkeit des Elastomerresonators abhängen.

Der Reflektionskoeffizient kann auch als Reflektionskoeffizienten bezeichnet sein. Der Reflektionskoeffizient repräsentiert dabei das Amplitudenverhältnis zwischen einer reflektierten Welle und einer einfallenden Welle. Insbesondere bezieht sich der Reflektionskoeffizient auf den Realanteil des zuvor genannten Amplitudenverhältnisses bei einer Resonanzfrequenz. Der Reflektionskoeffizient des Messresonators kann sich somit auf den Realanteil des Amplitudenverhältnisses zwischen einer reflektierten Welle und einer einfallenden Welle bei einer Resonanzfrequenz des Messresonators beziehen. In der Praxis wurde festgestellt, dass der Reflektionskoeffizient des Messresonators verändert wird, wenn sich die Permittivität des ersten Elastomermaterials und/oder die elektrische Leitfähigkeit des Elastomerresonators ändern. So verschiebt sich die für den Reflektionskoeffizienten relevante Resonanzfrequenz des Messresonators beispielsweise zu einer kleineren Frequenz, wenn die Permittivität des ersten Elastomermaterials und/oder die elektrische Leitfähigkeit des Elastomerresonators jeweils oder gemeinsam erhöhen.

Das System weist außerdem einen Transceiver auf. Der Transceiver ist vorzugsweise zum Erzeugen und Senden einer elektromagnetischen Welle und zum Empfangen einer elektromagnetischen Welle ausgebildet. Mit der Erzeugung der elektromagnetischen Welle ist der Transceiver vorzugsweise auch zur Erzeugung eines elektromagnetischen Wechselfelds ausgebildet. Der Transceiver kann eine Signalerzeugungs- und Empfangseinheit sowie eine damit gekoppelte Antenne aufweisen. Die Antenne kann als Dipolantenne ausgebildet sein. Für das System ist es deshalb vorgesehen, dass der Transceiver zur Erzeugung eines elektromagnetischen Wechselfelds ausgebildet ist. Die Frequenz, die Signalstärke und/oder die Bandbreite des elektromagnetischen Wechselfelds kann bzw. können vorbestimmt sein. So kann der Transceiver beispielsweise zur Erzeugung eines elektromagnetischen Wechselfelds mit einer vorbestimmten Signalstärke innerhalb eines vorbestimmten Frequenzbandes ausgebildet sein. Die mittlere Frequenz des Frequenzbands kann beispielsweise 100 MHz sein.

Der Messresonator ist ausgebildet, in das von dem Transceiver erzeugte Wechselfeld eingekoppelt zu werden und dieses Wechselfeld derart zu verändern, so dass das daraus resultierende Wechselfeld eine durch den Reflektionskoeffizienten des Messresonators geprägte Resonanzfrequenz aufweist. Diese Resonanzfrequenz entspricht vorzugsweise der Resonanzfrequenz des Messresonators, für den der Reflektionskoeffizient des Messresonators von der Permittivität des ersten Elastomermaterials und von der elektrischen Leitfähigkeit des Elastomerresonator abhängt. Daraus resultiert eine Abhängigkeit der Resonanzfrequenz des veränderten Wechselfelds von der Permittivität des ersten Elastomermaterials und von der elektrischen Leitfähigkeit des Elastomerresonators. Diese Abhängigkeit ist besonders robust gegenüber äußeren Störgrößen. Außerdem kann es ausreichen, wenn das Wechselfeld den Elastomerresonator nur schwach durchdringt. Die Verschiebung der Resonanzfrequenz des Messresonators in Abhängigkeit der genannten Permittivität und/oder Leitfähigkeit kann somit besonders störgrößenrobust erfolgen. Die Permittivität des ersten Elastomermaterials und die elektrische Leitfähigkeit des Elastomerresonators hängen wiederum von einem Alter des Elastomerprodukts und/oder einer mechanischen Belastung des Elastomerprodukts ab.

Der Transceiver ist zur Erfassung des Wechselfelds ausgebildet, dass von dem Messresonator verändert sein kann. Dieses Wechselfeld kann auch als das veränderte und/oder erfasste Wechselfeld bezeichnet sein. Außerdem ist der Transceiver zur Erzeugung eines Messsignals ausgebildet, dass das erfasste Wechselfeld und/oder die Resonanzfrequenz des erfassten Wechselfelds repräsentiert. Somit kann bereits der Transceiver in einer vorteilhaften Ausgestaltung dazu ausgebildet sein, die Resonanzfrequenz des erfassten Wechselfelds zu ermitteln. Darüber hinaus kann der Transceiver derart mit der Auswerteeinheit gekoppelt sein, um das Messsignal von dem Transceiver an die Auswerteeinheit zu übertragen.

Die Auswerteeinheit ist zur Ermittlung eines Alterungszustands des Elastomerprodukts basierend auf dem Messsignal ausgebildet. So kann die Auswerteeinheit dazu ausgebildet sein, den Alterungszustand basierend auf der von dem Messsignal repräsentierten Resonanzfrequenzen des erfassten Wechselfelds zu ermitteln. Denn wie zuvor erläutert, entspricht die Resonanzfrequenz des erfassten Wechselfelds vorzugsweise der Resonanzfrequenz des Messresonators, die wiederum von der Permittivität des ersten Elastomermaterials und der Leitfähigkeit des Elastomerresonators abhängt. Der Alterungszustand kann von der Permittivität des ersten Elastomermaterials und/oder von der elektrischen Leitfähigkeit des Elastomerresonators abhängen, oder umgekehrt. Somit besteht kann auch eine funktionale Abhängigkeit zwischen dem Alterungszustand des Elastomerprodukts und der Resonanzfrequenz des erfassten Wechselfelds bestehen. Dieser funktionale Zusammenhang kann von der Auswerteeinheit durch einen Datensatz und/oder eine mathematische Formel gespeichert sein und/oder zur Ermittlung des Alterungszustands verwendet werden.

Alternativ oder ergänzend ist es auch möglich, dass die Auswerteeinheit ausgebildet ist, den Alterungszustand basierend auf dem von dem Messsignal repräsentierten, erfassten Wechselfeld und/oder einer anderen direkt oder indirekt aus dem Wechselfeld ermittelbaren Größe zu ermitteln. Ein entsprechender, funktionaler Zusammenhang kann von der Auswerteeinheit durch einen Datensatz und/oder eine mathematische Formel gespeichert sein und/oder zur Ermittlung des Alterungszustands verwendet werden.

Der Alterungszustand kann ein Alterungswert sein. Der Alterungszustand bzw. der Alterungswert kann ein Alter und/oder eine mechanische Belastung des Elastomerprodukts repräsentieren. Der Alterungszustand kann von der Permittivität des ersten Elastomermaterials und/oder von der elektrischen Leitfähigkeit des Elastomerresonators abhängen, oder umgekehrt. Denn mit zunehmendem Alter des Elastomerprodukts verändern sich die Permittivität des Elastomermaterials und/oder die elektrische Leitfähigkeit des Elastomerresonators. Durch ein zunehmendes Alter und/oder durch eine mechanische Belastung des Elastomerprodukts verändert sich also die Permittivität des ersten Elastomermaterials und vorzugsweise die elektrische Leitfähigkeit des Elastomerresonators. Ein entsprechender Messresonator weist deshalb einen von dem Alter bzw. von der mechanischen Belastung abhängigen Reflektionskoeffizienten auf, der zu einer entsprechenden Prägung der Resonanzfrequenz des von dem Transceiver erfassten Wechselfelds führt. Durch die Erfassung des Wechselfelds kann deshalb mittels des Transceivers und/oder die Auswerteeinheit auf die Resonanzfrequenz des erfassten Wechselfelds geschlossen werden. Über die Resonanzfrequenz kann mittels der Auswerteeinheit auf den Alterungszustand des Elastomerprodukts geschlossen werden.

In der Praxis wurde festgestellt, dass die Permittivität des ersten Elastomermaterials und/oder die elektrische Leitfähigkeit des Elastomerresonators einen großen Einfluss auf den Reflektionskoeffizienten und somit auf die von dem Messresonator verursachte Verschiebung der Resonanzfrequenz des Wechselfelds haben. Die Auswirkung ist dabei nicht zuletzt deshalb besonders groß, da der Messresonator den Metallresonator aufweist, der in Verbindung mit dem zuvor erläuterten Teil des erstes Elastomermaterials und über dieses Teil des Elastomermaterials indirekt mit dem Elastomerresonator gekoppelt ist. Der daraus gebildete Messresonator kann deshalb eine besonders große Verschiebung der Resonanzfrequenz in Abhängigkeit der Permittivität des ersten Elastomermaterials und der elektrischen Leitfähigkeit des Elastomerresonators verursachen. Da sowohl die Permittivität des ersten Elastomermaterials als auch die elektrische Leitfähigkeit des Elastomerresonators den Alterungszustand des Elastomerprodukts indizieren, ist eine Ermittlung des Alterungszustands mittels des Elastomerprodukts des Systems sowie dem Receiver und der Auswerteeinheit des Systems besonders vorteilhaft und zugleich robust gegenüber möglichen Störgrößen möglich.

Eine vorteilhafte Ausgestaltung des Systems zeichnet sich dadurch aus, dass der Elastomerresonator zumindest im Wesentlichen mittig im ersten Elastomermaterial angeordnet ist. Eine mechanische Belastung des Elastomerprodukts, wie beispielsweise eine Biegung oder Streckung des Elastomerprodukts, führt deshalb bevorzugt zu einer mittleren Belastung des Elastomerresonators, die der mittleren Belastung des Elastomerprodukts entspricht. Die wiederum bietet den Vorteil, dass eine besonders präzise Ermittlung des Alterungszustands ermöglicht wird.

Eine weitere vorteilhafte Ausgestaltung des Systems zeichnet sich dadurch aus, dass der Metallresonator als ein Dipol-Metallresonator ausgebildet ist. Vorzugsweise ist der Metallresonator als eine resonante Schmalbandantenne oder als eine resonante Schmalbanddipolantenne ausgebildet. Vorzugsweise weist der Metallresonator eine gestreckte, längliche Form auf. So kann der Metallresonator durch einen gestreckten Metallstab und/oder einen gestreckten Metallstreifen gebildet sein. Besonders bevorzugt ist der Metallresonator als eine metallische Dipolantenne ausgebildet. Der Metallresonator weist vorzugsweise eine Einspeisebandbreite auf, die kleiner als 20 MHz oder kleiner als 30 MHz ist. Besonders bevorzugt weist der Metallresonator eine Einspeisebandbreite von weniger als 10 MHz auf. Durch die besonders kleine Einspeisebandbreite des Metallresonators kann zusammen mit dem Elastomerresonator und dem Teil des ersten Elastomermaterials, der zwischen dem Metallresonator und dem Elastomerresonator angeordnet ist, eine vorteilhafte Ausgestaltung für den Messresonator gewährleistet werden, der ebenfalls eine besonders geringe Einspeisebandbreite aufweist, und außerdem gewährleistet, dass die Permittivität des ersten Elastomermaterials und die elektrische Leitfähigkeit des Elastomerresonators hohen Einfluss auf die Verschiebung des Reflektionskoeffizienten hat, was wiederum zu einer entsprechenden Prägung der Resonanzfrequenz des Wechselfelds führt, wenn der Messresonator zur Ankopplung mit dem Wechselfeld gebracht wird.

Eine vorteilhafte Ausgestaltung des Systems zeichnet sich dadurch aus, dass der Elastomerresonator in einem vorbestimmten Abstand zwischen 1 mm und 10 mm zumindest im Wesentlichen parallel zu dem Metallresonator angeordnet ist. Der zuvor genannte, vorbestimmte Abstand kann insbesondere derart gewählt werden, dass die Leitfähigkeit des Elastomerresonators einen besonders großen Einfluss auf den Reflektionskoeffizienten des Messresonators hat. So kann der vorbestimmte Abstand zwischen dem Elastomerresonator und dem Metallresonator auf einen Wert von kleiner als 5 mm bestimmt sein. Außerdem ist es bevorzugt vorgesehen, dass der Elastomerresonator parallel zu dem Metallresonator angeordnet ist. Dadurch kann eine besonders symmetrische Beeinflussung des Wechselfelds erfolgen, wenn dieses beispielsweise durch den Transceiver derart hervorgerufen wird, dass das Wechselfeld im Wesentlichen symmetrisch auf den Metallresonator wirkt.

Eine weitere vorteilhafte Ausgestaltung des Systems zeichnet sich dadurch aus, dass der Transceiver zur Erzeugung eines elektromagnetischen Wechselfelds mit einer mittleren Basisfrequenz zwischen 80 Mhz und 120 Mhz ausgebildet ist. Die Basisfrequenz kann beispielsweise die mittlere Frequenz der von dem Transceiver erzeugbaren, elektromagnetischen Wellen sein. Der Transceiver kann beispielsweise zur Erzeugung von elektromagnetischen Wellen mit Frequenzen zwischen 80 Mhz und 120 Mhz ausgebildet sein. Die Basisfrequenz wäre in diesem Fall 100 MHz. In der Praxis wurde festgestellt, dass der zuvor genannte Frequenzbereich besonders vorteilhaft zur Wechselwirkung mit dem Messresonator geeignet ist. Die Resonanzfrequenz des Messresonators kann in dem in Frequenzbereich der von dem Transceiver erzeugbaren Wellen sein. Deshalb kann mit dem zuvor genannten Frequenzbereich auch eine besonders präzise Erfassung des Alterungszustands des Elastomerprodukts erreicht werden.

Eine weitere vorteilhafte Ausgestaltung des Systems zeichnet sich dadurch aus, dass die Auswerteeinheit ausgebildet ist, die Resonanzfrequenz des erfassten Wechselfelds basierend auf dem Messsignal zu ermitteln. Die Auswerteeinheit kann außerdem ausgebildet sein, den Alterungszustands des Elastomerprodukts basierend oder außerdem basierend auf der Resonanzfrequenz zu ermitteln. So kann die Auswerteeinheit die Resonanzfrequenz beispielsweise aus dem erfassten Wechselfeld extrahieren und/oder ermitteln, und zwar basierend auf dem Messsignal, dass die Auswerteeinheit von dem Transceiver übermittelt bekommt. Außerdem kann die Auswerteeinheit ausgebildet sein, den Alterungszustand ausschließlich mittels der ermittelten Resonanzfrequenz oder außerdem mit Hilfe der ermittelten Resonanzfrequenz zu ermitteln. Die Resonanzfrequenz ist dabei die Resonanzfrequenz des erfassten Wechselfelds. Sie korrespondiert aber vorzugsweise auch zu der Resonanzfrequenz des Messresonators. Die Berücksichtigung der ermittelten Resonanzfrequenz bei der Ermittlung des Alterungszustands hat sich in der Praxis als besonders vorteilhaft erwiesen, um eine präzise Ermittlung des Alterungszustands des Elastomerprodukts zu ermöglichen. Außerdem kann die Resonanzfrequenz zumindest im Wesentlichen und/oder indirekt von der Permittivität des ersten Elastomermaterials und der Leitfähigkeit des Elastomerresonators abhängen. Weitere äußere Einflüsse können demgegenüber zurücktreten, so dass die Ermittlung des Alterungszustands des Elastomerprodukts zumindest außerdem basieren oder ausschließlich basierend auf der Resonanzfrequenz besonders störgrößenrobust ist.

Eine weitere vorteilhafte Ausgestaltung des Systems zeichnet sich dadurch aus, dass von der Auswerteeinheit eine Referenzfrequenz gespeichert ist, wobei die Auswerteeinheit ausgebildet ist, den Alterungszustand des Elastomerprodukts außerdem basierend auf der Referenzfrequenz zu ermitteln. Die Auswerteeinheit kann dazu ausgebildet sein, eine Verschiebung der Resonanzfrequenz relativ zu der Referenzfrequenz zu ermitteln, und diese Verschiebung der Resonanzfrequenz bei der Ermittlung des Alterungszustands des Elastomerprodukts zu berücksichtigen oder die Ermittlung des Alterungszustands des Elastomerprodukts ausschließlich auf dieser Verschiebung der Resonanzfrequenz auszuführen. Somit bietet die Referenzfrequenz einen Ausgangspunkt, der beispielsweise einen Wert für die Resonanzfrequenz eines neuwertigen Elastomerprodukts repräsentiert. Mit anderen Worten kann die Referenzfrequenz den Wert der Resonanzfrequenz des Wechselfelds repräsentieren, wenn das neuwertige Elastomerprodukt mit dem entsprechend ausgebildeten Messresonator in das Wechselfeld gebracht wird, so dass der Messresonator in Wechselwirkung mit dem Wechselfeld tritt.

Eine weitere vorteilhafte Ausgestaltung des Systems zeichnet sich dadurch aus, dass das erste Elastomermaterial elektrisch isolierend ausgebildet ist. Dies verhindert insbesondere, dass es zu einem elektrischen Kurzschluss zwischen dem Metallresonator und dem Elastomerresonator kommt. Denn beide sind elektrisch leitfähig. Das elektrisch isolierend ausgebildete, erste Elastomermaterial kann also als Isolator zwischen den Metallresonator und dem Elastomerresonator wirken. Dazu kann das erste Elastomermaterial beispielsweise einen spezifischen Widerstand von mindestens 10¹⁴ Ω^{∗}cm aufweisen.

Eine vorteilhafte Ausgestaltung des Systems zeichnet sich dadurch aus, dass das erste Elastomermaterial als ein erstes Gummimaterial ausgebildet ist.

Eine weitere vorteilhafte Ausgestaltung des Systems zeichnet sich dadurch aus, dass das zweite Elastomermaterial als ein zweites, elektrisch leitfähiges Gummimaterial ausgebildet ist. Dies ist insbesondere dann von Vorteil, wenn das erste Elastomermaterial als ein erstes Gummimaterial ausgebildet ist. Denn in diesem Fall können das erste Gummimaterial und das zweite Gummimaterial eine besonders feste Verbindung zueinander aufweisen, insbesondere eine stoffschlüssige Verbindung zueinander aufweisen. Eine mechanische Belastung des Gummiprodukts kann sich somit in entsprechender Weise auf das zweite Gummimaterial auswirken. Damit lässt sich der Alterungszustand des Elastomerprodukts besonders präzise bestimmen. Sind das erste Elastomermaterial als das erste Gummimaterial und das zweite Elastomermaterial als das zweite Gummimaterial ausgebildet, so kann das Elastomerprodukt auch als ein Gummiprodukt bezeichnet sein. Ist das erste Elastomermaterial als das erste Gummimaterial ausgebildet, so kann das erste Elastomermaterial auch als das erste Gummimaterial bezeichnet sein. Ist das zweite Elastomermaterial als das zweite Gummimaterial ausgebildet, so kann das zweite Elastomermaterial auch als das zweite Gummimaterial bezeichnet sein.

Eine vorteilhafte Ausgestaltung des Systems zeichnet sich dadurch aus, dass der Elastomerresonator passiv ausgebildet ist. Dem Elastomerresonator sind in diesem Fall keine elektrischen, weiteren Bauteile, insbesondere keine weiteren, aktiven Bauteile, zugeordnet. Vielmehr kann es vorgesehen sein, dass der Elastomerresonator ausschließlich von dem zweiten Elastomermaterial gebildet ist. Damit kann der Elastomerresonator besonders vorteilhaft in das erste Elastomermaterial des Elastomerprodukts eingebettet sein, ohne die mechanischen Eigenschaften des Elastomerprodukts wesentlich zu beeinflussen. Denn sowohl das erste Elastomermaterial als auch der Elastomerresonator in dieser vorteilhaften Ausgestaltung können jeweils formfeste, aber elastisch verformbare Teile des Elastomerprodukts bilden.

Eine vorteilhafte Ausgestaltung des Systems zeichnet sich dadurch aus, dass der Metallresonator rein passiv ausgebildet ist. So kann der Metallresonator beispielsweise durch eine Dipol-Metallantenne ausgebildet sein, die mit keinen weiteren Bauteilen elektrisch gekoppelt ist. Der Metallresonator ist in diesem Fall besonders robust gegenüber mechanischen Verformungen des Elastomerprodukts. So kann der Metallresonator derart angeordnet und/oder ausgebildet sein, um elastisch verformt zu werden.

Eine weitere vorteilhafte Ausgestaltung des Systems zeichnet sich dadurch aus, dass das System eine mit der Auswerteeinheit gekoppelte Signalschnittstelle zur Übertragung eines Statussignals aufweist, dass dem von der Auswerteeinheit ermittelbaren Alterungszustand des Elastomerprodukts repräsentiert. Das Statussignal kann von der Auswerteeinheit erzeugt werden, so dass das Statussignal einen von der Auswerteeinheit ermittelten Alterungszustand des Elastomerprodukts repräsentiert. Die Signalschnittstelle kann direkt oder indirekt mit der Auswerteeinheit gekoppelt sein. Es ist aber auch möglich, dass die Signalschnittstelle einen Teil der Auswerteeinheit bildet. Die Auswerteeinheit kann die gekoppelte Signalschnittstelle derart steuern, dass das Statussignal mittels der Signalschnittstelle gesendet wird, beispielsweise an einer übergeordneten Signalempfangseinheit, die zur Weiterverarbeitung des Statussignals ausgebildet ist.

Eine weitere vorteilhafte Ausgestaltung des Systems zeichnet sich dadurch aus, dass das Elastomerprodukt ein Reifen, eine Luftfeder, ein Gummilager, ein Förderband, ein Riemen oder ein Schlauch ist.

Weitere Merkmale, Vorteile und Anwendungsmöglichkeiten der vorliegenden Erfindung ergeben sich aus der nachfolgenden Beschreibung der Ausführungsbeispiele und den Figuren. Dabei bilden alle beschriebenen und/oder bildlich dargestellten Merkmale für sich und in beliebiger Kombination den Gegenstand der Erfindung auch unabhängig von ihrer Zusammensetzung in den einzelnen Ansprüchen oder deren Rückbezügen. In den Figuren stehen weiterhin gleiche Bezugszeichen für gleiche oder ähnliche Objekte.
- Figur 1: zeigt eine vorteilhafte Ausgestaltung des Systems in einer schematischen Ansicht.
- Figur 2: zeigt ein Diagramm mit mehreren Kennlinien für den Reflektionskoeffizienten.

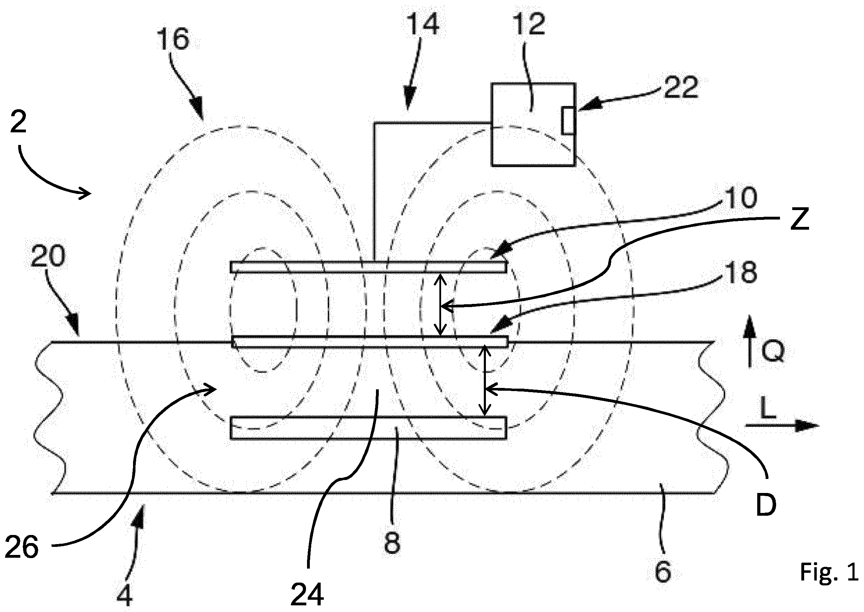

In der Figur 1 ist eine vorteilhafte Ausgestaltung des Systems 2 schematisch dargestellt. Das System 2 weist ein Elastomerprodukt 4, einen Transceiver 10 und eine Auswerteeinheit 12 auf. Vorzugsweise ist der Transceiver 10 über eine Signalleitung 14 mit der Auswerteeinheit 12 gekoppelt. Der Transceiver 10 ist außerdem bevorzugt in einem Abstand Z zu dem Elastomerprodukt 4 angeordnet, so dass der Transceiver 10 das Elastomerprodukt 4 nicht berührt. Mit anderen Worten kann der Transceiver 10 berührungsfrei zu dem Elastomerprodukt 4 angeordnet sein.

Das Elastomerprodukt 4 weist ein erstes Elastomermaterial 6 auf, in das ein Elastomerresonator 8 eingebettet ist. Bei dem ersten Elastomermaterial 6 handelt es sich vorzugsweise um ein erstes Gummimaterial. Außerdem ist das erste Elastomermaterial 6 vorzugsweise elektrisch isolierend ausgebildet. Der Elastomerresonator 8 ist vorzugsweise vollständig von einem zweiten, elektrisch leitfähigen Elastomermaterial gebildet. Hierbei handelt es sich vorzugsweise um ein elektrisch leitfähiges, zweites Gummimaterial. Das Elastomerprodukt 4 weist beispielsweise eine Höhe in Höhenrichtung Q des Elastomerprodukts 4 und eine vorbestimmte Länge in Längsrichtung des Elastomerprodukts auf, so dass der Elastomerresonator als Streifen ausgebildet ist.

Der Elastomerresonator 8 kann mittig zwischen den beiden in der Höhenrichtung Q gegenüberliegenden Außenseiten 20 angeordnet. Dadurch kann besonders einfach gewährleistet werden, dass der Elastomerresonator 8 vollständig von dem ersten Elastomermaterial 6 des ersten Elastomerprodukts 4 umgeben ist. Die Außenfläche des Elastomerresonators 8 ist vorzugsweise vollständig in Kontakt mit dem ersten Elastomermaterial 6. Dadurch kann besonderes effektiv gewährleistet werden, dass eine mechanische Verformung des Elastomerresonators 8 zu einer entsprechenden, mechanischen Verformung des ersten Elastomermaterials 6 führt. Der Alterungszustand des Elastomerprodukts 4 kann somit zu dem Alterungszustand des zugehörigen Elastomerresonators 8 korrespondieren.

Das Elastomerprodukt 4 weist außerdem einen Metallresonator 18 auf. Der Metallresonator 18 ist vorzugsweise von einer metallischen Dipolantenne gebildet. Die Dipolantenne kann eine stabförmige oder streifenförmige Form aufweisen. Wie aus der Figur 1 beispielhaft zu entnehmen ist, ist der Metallresonator 18 an einer Außenseite 20 des Elastomerprodukts 4 angeordnet. So kann der Metallresonator 18 beispielsweise auf der Außenseite 20 angeordnet sein und mit dem ersten Elastomermaterial 6 stoffschlüssig verbunden sein. Es ist aber auch möglich, dass der Metallresonator 18 zumindest teilweise in das erste Elastomermaterial 6 einfasst. Dadurch kann außerdem eine formschlüssige Verbindung zwischen dem Metallresonator 18 und dem ersten Elastomermaterial 6 des Elastomerprodukts 4 gewährleistet werden. Zumindest ein Teil des Metallresonators 18 bildet jedoch einen Teil der Außenfläche des Elastomerprodukts 4.

Außerdem ist es vorgesehen, dass der in das erste Elastomermaterial 6 eingebettete Elastomerresonator 8 beabstandet zu dem Metallresonator 18 angeordnet ist. Der Abstand D zwischen dem Elastomerresonator 8 und dem Metallresonator 18 kann vorbestimmt sein. Besonders bevorzugt ist es vorgesehen, dass der genannte Abstand D beispielsweise in einem Bereich zwischen 0,2 mm und 15 mm ist. Ein anderer geeigneter Abstand D zwischen dem Elastomerresonator 8 und dem Metallresonator 18 kann ebenfalls vorgesehen sein. Es ist jedoch vorgesehen, dass der Elastomerresonator 8 und der Metallresonator 18 keinen unmittelbaren Kontakt zu einander aufweisen. Vielmehr ist ein Teil 24 des ersten Elastomermaterials 6 zwischen dem Elastomerresonator 8 und dem Metallresonator 18 angeordnet. Außerdem ist es bevorzugt vorgesehen, dass der Elastomerresonator 8 und der Metallresonator 18 zumindest im Wesentlichen parallel zueinander angeordnet sind.

Der Metallresonator 18, der Elastomerresonator 8 und der Teil 24 des ersten Elastomermaterials 6, das zwischen dem Elastomerresonator 8 und dem Metallresonator 18 angeordnet ist, bilden zusammen einen sogenannten Messresonator. Hierbei handelt es sich ebenfalls um einen Resonator, der von den zuvor genannten Resonatoren 18, 8 und dem genannten Teil 24 gebildet ist. An dieser Stelle soll erwähnt werden, dass jeder der genannten Resonatoren 18, 8, 26 vorzugsweise als ein jeweiliger, elektromagnetischer Resonator ausgebildet ist. Dabei weisen die Resonatoren 18, 8, 26 jeweils vorzugsweise keinen Kondensator und keine Spule auf.

Wird der Metallresonator 18 separat betrachtet, so kann der Metallresonator 18 derart ausgebildet, dass der Metallresonator 18 bei einer breitbandigen Anregung mit einem elektromagnetischen Feld mit der zugehörigen Resonanzfrequenz schwingt.

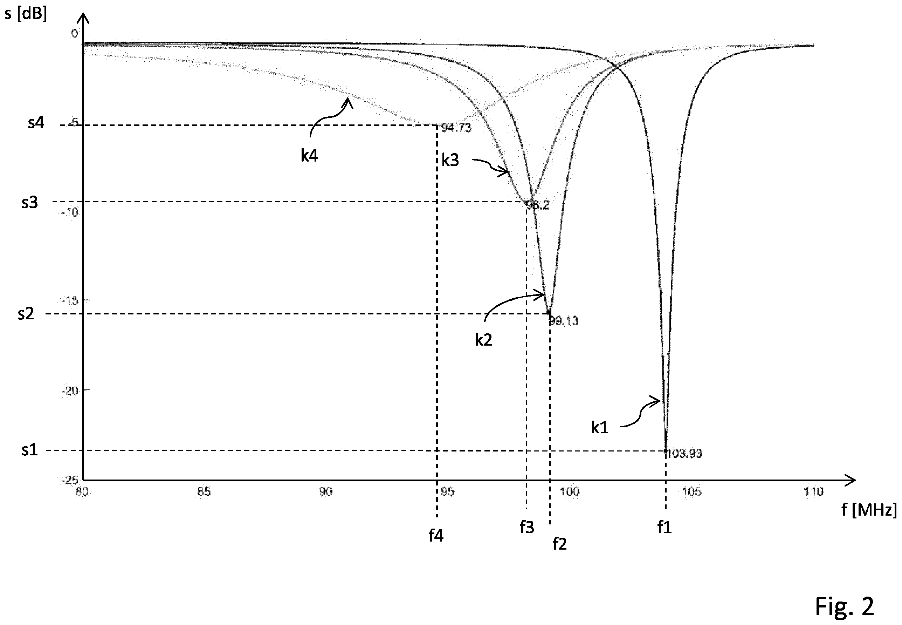

In der Figur 2 ist ein Diagramm dargestellt, dass den Reflektionskoeffizienten s [dB] gegenüber der Frequenz f ^{┌}MHz^{┐} wiedergibt. Die in dem Diagramm dargestellte Kennlinie k1 repräsentiert beispielhaft den Reflektionskoeffizienten s des Metallresonators 18 für verschiedene Frequenzen f im Bereich von 80 MHz bis 110 MHz. Aus dem Verlauf der Kennlinie k1 des Reflektionskoeffizienten kann entnommen werden, dass die Resonanzfrequenz f1 des Metallresonators 18 etwa 103 MHz beträgt und hier der Wert des Reflektionskoeffizienten s1 etwa - 23,5 dB ist.

Wird der Metallresonator 18, wie beispielhaft in der Figur 1 dargestellt, an der Außenseite 20 des Elastomerprodukts 4 angeordnet, so dass der Metallresonator 18, der Elastomerresonator 8 und der Teil 24 des ersten Elastomermaterials 6 den zuvor erläuterten Messresonator 26 bilden, so weist der Messresonator 26 einen Verlauf der Kennlinie k4 des Reflektionskoeffizienten auf, der ebenfalls beispielhaft aus dem Diagramm der Figur 2 zu entnehmen ist. Rein beispielhaft beträgt die Resonanzfrequenz f4 des Metallresonators 26 ausgehend von der Kennlinie k4 des Reflektionskoeffizienten für diesen Messresonator 26 etwa 94 MHz und der zugehörige Reflektionskoeffizient s2 beträgt etwa - 5 dB.

In der Praxis wurde festgestellt, dass die Anordnung des elektrisch leitfähigen Elastomerresonators 8 und des Teils 24 des vorzugsweise elektrisch isolierenden, ersten Elastomermaterials 6 zwischen dem Elastomerresonator 8 und dem Metallresonator 18 zu einer Verformung der Kennlinie des Reflektionskoeffizienten des Messresonators 26 führen. Daraus resultiert, dass die elektrische Leitfähigkeit des Elastomerresonators 8 und die Permittivität des Teils 24 des ersten Elastomermaterials 6 zu einer Verschiebung der Resonanzfrequenz des Messresonators 26 führen. Darüber hinaus kommt es bei der Verschiebung der Resonanzfrequenz von beispielsweise f4 zu f3 zu einer entsprechenden Verschiebung der für die jeweilige Resonanzfrequenz relevanten Wert des Reflektionskoeffizienten von s4 zu s3. Bei dem Reflektionskoeffizienten des Messresonators 26 ist vorzugsweise immer der Wert des Reflektionskoeffizienten s bei der jeweiligen Resonanzfrequenz des Messresonators 26 gemeint. Insbesondere ist mit dem Reflektionskoeffizienten auch der realteilige Reflektionskoeffizient gemeint.

Aus der Zusammenschau der Verschiebung der Resonanzfrequenz von f4 zu f3 durch die zuvor erläuterte Anordnung des Metallresonators 18 an der Außenseite 20 des Elastomerprodukts 4, so dass der Elastomerresonator 8 zumindest im Wesentlichen gegenüberliegend zu dem Metallresonator 18 angeordnet ist und der Teil 24 des ersten Elastomermaterials 6 zwischen dem Elastomerresonator 8 und Metallresonator 18 angeordnet ist, lässt sich schließen, dass der Reflektionskoeffizient s des Messresonators 26 von der Permittivität des Teils 24 des ersten Elastomermaterials 6 und von der elektrischen Leitfähigkeit des Elastomerresonators 8 abhängt. Entsprechendes gilt vorzugsweise für die Resonanzfrequenz f des Messresonators 26. Denn die Resonanzfrequenz f hängt vorzugsweise jedenfalls von der Permittivität des Teils 24 des ersten Elastomermaterials 6 und von der elektrischen Leitfähigkeit des Elastomerresonators 8 ab.

Wie aus der Figur 1 beispielhaft zu entnehmen ist, ist der Transceiver 10 zur Erzeugung eines elektromagnetischen Wechselfelds 16 ausgebildet. Das Wechselfeld 16 ist schematisch durch die gestrichelten Linien. Der Transceiver 10 kann zur Erzeugung von elektromagnetischen Wellen ausgebildet sein, die beispielsweise einen Frequenzbereich von 80 MHz bis 120 MHz umfassen. Außerdem kann der Transceiver 10 zur Erfassung von elektromagnetischen Wellen und somit zur Erfassung eines veränderten, elektromagnetischen Wechselfelds 16 ausgebildet sein. Vorzugsweise ist der Transceiver 10 über die Signalleitung 14 mit der Auswerteeinheit 12 gekoppelt. Der Transceiver 10 ist außerdem zur Erzeugung eines Messsignals ausgebildet, dass das erfasste Wechselfeld 16 repräsentiert. Schließlich kann der Transceiver 10 ausgebildet sein, das Messsignal über die Signalleitung 14 an die Auswerteeinheit 12 zu übertragen.

Der Messresonator 26 ist ausgebildet, in das von dem Transceiver 10 erzeugte Wechselfeld 16 eingekoppelt zu werden. Dabei ist es bevorzugt vorgesehen, dass die Resonanzfrequenz f des Messresonators 26 innerhalb einer Frequenzbandbreite des von dem Transceiver 10 erzeugten elektromagnetischen Wechselfelds 16 ist. Wird von dem Transceiver 10 beispielsweise ein elektromagnetisches Wechselfeld 16 mit einer Bandbreite zwischen 80 MHz und 120 MHz erzeugt, so kann der Messresonator 26, insbesondere durch die Wahl des Metallresonators 18 und/oder des Elastomerresonators 8, derart ausgebildet sein, dass die Resonanzfrequenz f4 des Messresonators 26 zwischen 80 MHz und 120 MHz, insbesondere zwischen 90 MHz und 110 MHz ist. Denn in diesem Fall kann der Messresonator 26 durch das von dem Transceiver 10 erzeugte elektromagnetische Wechselfeld 16 zur Schwingung angeregt werden, was zu einer entsprechenden Veränderung des Wechselfelds 16 führt. Mit anderen Worten ist der Messresonator 26 zur Veränderung des Wechselfelds 16 derart ausgebildet, so dass das veränderte Wechselfeld 16 eine durch den Reflektionskoeffizienten s4 des Messresonators 26 geprägte Resonanzfrequenz f4 aufweist. Denn durch die Einkopplung des Messresonators 26 wird das Wechselfeld 16, insbesondere durch die dem Messresonator 26 zugeordnete Resonanzfrequenz f4, verändert, so dass aus dem Frequenzgang des erfassten und/oder veränderten Wechselfelds 16 die dem Messresonator 26 zugeordnete Resonanzfrequenz f4 ablesbar und/oder daraus ermittelbar ist. Hierzu kann der Transceiver 10 und/oder die Auswerteeinheit 12 ausgebildet sein.

Die Resonanzfrequenz f4 des Messresonators 26 hängt von der elektrischen Leitfähigkeit des Elastomerresonators und von der Permittivität des Teils 24 des ersten Elastomermaterials 6 ab. Durch eine mechanische Belastung des Elastomerprodukts 4, beispielsweise durch Biegung und/oder Streckung, sowie mit zunehmendem Alter des Elastomerprodukts 4 verändern sich die elektrische Leitfähigkeit des Elastomerresonators 8 und die Permittivität des Teils 24 des ersten Elastomermaterials 6. Insbesondere verringert sich die zuvor genannte Permittivität des Teils 24 des ersten Elastomermaterials 6 sowie die elektrische Leitfähigkeit des Elastomerresonators 8 aufgrund der mechanischen Belastung und/oder aufgrund des zunehmenden Alters des Elastomerprodukts 4. Weist der Messresonator 26 eines neuwertigen Elastomerprodukts 4 beispielsweise die in Figur 2 dargestellte Resonanzfrequenz f4 und den zugehörigen Reflektionskoeffizient s4 auf, so kann sich die Resonanzfrequenz mit zunehmendem Alter und/oder mit zunehmender Belastung des Elastomerprodukts 4 zu der Frequenz f3 oder zu einer Frequenz f2 verschieben, was zu einem entsprechenden Reflektionskoeffizienten s3 bzw. s2 führt.

Unter einem Alterungszustand kann ein Wert, der als Alterungswert bezeichnet sein kann, verstanden werden, der ein Alter und/oder eine mechanische Belastung des Elastomerprodukts 4 repräsentiert. Aufgrund der zuvor erläuterten Verschiebung der Resonanzfrequenz des Messresonators 26 von f4 zu f3 bzw. f2 und/oder der entsprechenden Verschiebung Reflektionskoeffizienten des Messresonators 26 von s4 zu s3 bzw. s2 in Abhängigkeit des Alterungszustands des Messresonators 26, der gleichermaßen den Alterungszustand des Elastomerprodukts 4 repräsentiert kann, kann aus der durch den Messresonator 26 verursachten Veränderung des Wechselfelds 16 und der entsprechenden Prägung der Resonanzfrequenz des Wechselfelds 16 auf die entsprechende Resonanzfrequenz des Messresonators 26 und deshalb auch auf den Alterungszustand des Messresonators 26 und/oder des Elastomerprodukts 4 geschlossen werden. Hierzu kann die Auswerteeinheit 12 ausgebildet sein. Es ist deshalb vorgesehen, dass die Auswerteeinheit 12 zur Ermittlung eines Alterungszustands des Elastomerprodukts 4 basierend auf dem Messsignal des Transceivers 10 ausgebildet ist, dass das von dem Transceiver 10 erfasste Wechselfeld 16 und/oder die Resonanzfrequenz f2, f3, f4 des erfassten Wechselfelds 16 repräsentiert.

Von der Auswerteeinheit 12 kann ein Datensatz gespeichert sein, der den jeweiligen Alterungszustand für eine Vielzahl von Resonanzfrequenzen repräsentiert. Die Auswerteeinheit 12 kann unter Verwendung dieses Datensatzes und der aus dem erfassten Wechselfeld ermittelten Resonanzfrequenz den zuvor erläuterten Alterungszustand des Elastomerprodukts 4 ermitteln. Alternativ oder ergänzend zu dem Datensatz kann von der Auswerteeinheit 12 eine Abbildungsfunktion gespeichert sein, mittels der der Alterungszustand in Abhängigkeit der Resonanzfrequenz des erfassten Wechselfelds 16 ermittelbar ist. Die Auswerteeinheit 16 kann alternativ oder ergänzend die zuvor genannte Abbildungsfunktion verwenden, um den Alterungszustand des Elastomerprodukts 4 zu ermitteln.

Wie aus der Figur 1 beispielhaft zu entnehmen ist, ist es bevorzugt vorgesehen, dass das System 2 außerdem eine Signalschnittstelle 22 aufweist. Die Signalschnittstelle 22 kann mit der Auswerteeinheit 12 gekoppelt sein. Dies kann auch eine Ausgestaltung umfassen, bei der die Signalschnittstelle 22 einen Teil der Auswerteeinheit 12 bildet. Die Signalschnittstelle 22 ist zur Übertragung eines Statussignals ausgebildet. Das Statussignal kann von der Auswerteeinheit 12 ermittelt werden. Das Statussignal repräsentiert den von der Auswerteeinheit 12 ermittelten Alterungszustand des Elastomerprodukts 4. Mittels der Signalschnittstelle 22 kann also das Statussignal an eine andere Einheit übertragen werden, so dass dieser anderen Einheit die Information über den Alterungszustand des Elastomerprodukts 4 zugängig wird.

Außerdem ist es bevorzugt vorgesehen, dass der Metallresonator 18 und der Elastomerresonator 8 einander überlappend angeordnet sind, vorzugsweise derart, dass ein von dem Transceiver 10 erzeugtes, elektromagnetisches Wechselfeld 16 sowohl den Metallresonator 18 und dem Elastomerresonator 8 durchdringt.

Wie es beispielhaft aus der Figur 1 zu entnehmen ist, kann es bevorzugt sein, dass der Elastomerresonator 8 in Höhenrichtung Q mittig angeordnet ist. Eine Verformung durch Biegung und/oder Streckung des Elastomerprodukts 4 kann somit eine entsprechende Verformung des Elastomerresonators 8 hervorrufen, so dass mittels des Elastomerresonators 8 als einen Teil des Messresonators 26 eine besonders präzise Ermittlung des Alterungszustands des Elastomerprodukts 4 ermittelbar ist.

Außerdem ist es bevorzugt vorgesehen, dass der Elastomerresonator 8 rein passiv ausgebildet ist. So kann der Elastomerresonator 8 beispielsweise ausschließlich von dem elektrisch leitfähigen, zweiten Elastomermaterial gebildet sein. Außerdem kann es bevorzugt vorgesehen sein, dass dieses Elastomermaterial als ein zweites, elektrisch leitfähiges Gummimaterial ausgebildet ist.

Außerdem ist es bevorzugt vorgesehen, dass der Metallresonator 18 rein passiv ausgebildet ist. Der Metallresonator 18 kann beispielsweise durch einen Metallstrang, insbesondere in Form einer Stabantenne, ausgestaltet sein. Weitere elektrische Funktionsbauteile sind vorzugsweise nicht mit dem Metallresonator 18 gekoppelt.

Schließlich sei darauf hingewiesen, dass das Elastomerprodukt 4 beispielsweise als ein Fahrzeugreifen, eine Luftfeder, ein Gummilager, ein Förderband, ein Riemen oder als ein Schlauch ausgebildet sein kann. So kann der Metallresonator 18 beispielsweise an einer Außenseite eines Förderbands oder eines Riemens angeordnet sein. Entsprechendes gilt für jede der zuvor beispielhaft genannten Ausgestaltungen des Elastomerprodukts 4.

Ergänzend sei darauf hingewiesen, dass "aufweisend" keine anderen Elemente oder Schritte ausschließt und "ein" oder "eine" keine Vielzahl ausschließt. Ferner sei darauf hingewiesen, dass Merkmale, die mit Verweis auf eines der obigen Ausführungsbeispiele beschrieben worden sind, auch in Kombination mit anderen Merkmalen anderer oben beschriebener Ausführungsbeispiele verwendet werden können. Bezugszeichen in den Ansprüchen sind nicht als Einschränkung anzusehen.

### Bezugszeichenliste

- D: Abstand
- L: Längsrichtung
- Q: Höhenrichtung
- Z: Abstand
- k: Kennlinie eines Reflektionskoeffizienten
- s: Reflektionskoeffizient
- f: Frequenz

- 2: System
- 4: Elastomerprodukt
- 6: erste Elastomermaterial
- 8: Elastomerresonator
- 10: Transceiver
- 12: Auswerteeinheit
- 14: Signalleitung
- 16: Wechselfeld
- 18: Metallresonator
- 20: Außenseite
- 22: Signalschnittstelle
- 24: Teil des ersten Elastomermaterials
- 26: Messresonator

## Patentansprüche

1. System (2) zur Ermittlung eines Alterungszustands eines Elastomerprodukts (4), wobei das System (2) aufweist:
ein Elastomerprodukt (4) mit einem ersten Elastomermaterial (6),
einen Transceiver (10), und
eine Auswerteeinheit (12), die mit dem Transceiver (10) gekoppelt ist,
wobei das Elastomerprodukt (4) einen Elastomerresonator (8) und einen Metallresonator (18) aufweist,
wobei der Elastomerresonator (8) in das erste Elastomermaterial (6) des Elastomerprodukts (4) eingebettet ist,
wobei der Elastomerresonator (8) von einem Streifen aus einem zweiten, elektrisch leitfähigen Elastomermaterial ausgebildet ist,
wobei der Metallresonator (18) an einer Außenseite (20) des Elastomerprodukts (4) angeordnet ist, so dass der Metallresonator (18) und der Elastomerresonator (8) gegenüberliegend und kontaktfrei zueinander angeordnet sind,
wobei der Metallresonator (18), der Elastomerresonator (8) und ein Teil (24) des ersten Elastomermaterial (6), der zwischen dem Metallresonator (18) und dem Elastomerresonator (8) angeordnet ist, einen Messresonator (26) bilden, dessen Reflektionskoeffizient (s2, s3, s4) von der Permittivität des ersten Elastomermaterials (6) und von der elektrischen Leitfähigkeit des Elastomerresonators (8) abhängt,
wobei der Transceiver (10) zur Erzeugung eines elektromagnetischen Wechselfelds (16) ausgebildet ist,
wobei der Messresonator (26) ausgebildet ist, in das Wechselfeld (16) eingekoppelt zu werden und das Wechselfeld (16) derart zu verändern, so dass das Wechselfeld (16) eine durch den Reflektionskoeffizienten (s2, s3, s4) des Messresonators (26) geprägte Resonanzfrequenz (f2, f3, f4) aufweist,
wobei der Transceiver (10) zur Erfassung des Wechselfelds (16) und zur Erzeugung eines Messsignals ausgebildet ist, das das Wechselfeld (16) und/oder die Resonanzfrequenz (f2, f3, f4) des Wechselfelds (16) repräsentiert, und
wobei die Auswerteeinheit (12) zur Ermittlung eines Alterungszustands des Elastomerprodukts (4) basierend auf dem Messsignal ausgebildet ist.

2. System (2) nach dem vorhergehenden Anspruch, **dadurch gekennzeichnet, dass** der Elastomerresonator (8) zumindest im Wesentlichen mittig im ersten Elastomermaterial (6) angeordnet ist.

3. System (2) nach einem der vorhergehenden Ansprüche, **dadurch gekennzeichnet, dass** der Metallresonator (18) als ein Dipol-Metallresonator ausgebildet ist.

4. System (2) nach einem der vorhergehenden Ansprüche, **dadurch gekennzeichnet, dass** der Elastomerresonator (8) in einem vorbestimmten Abstand D zwischen 1 mm und 10 mm zumindest im Wesentlichen parallel zu dem Metallresonator (18) angeordnet ist.

5. System (2) nach einem der vorhergehenden Ansprüche, **dadurch gekennzeichnet, dass** der Transceiver (10) zur Erzeugung eines elektromagnetischen Wechselfelds (16) mit einer mittleren Basisfrequenz zwischen 80 MHz und 120 MHz ausgebildet ist.

6. System (2) nach einem der vorhergehenden Ansprüche, **dadurch gekennzeichnet, dass** die Auswerteeinheit (12) ausgebildet ist, die Resonanzfrequenz (f2, f3, f4) des Wechselfelds (16) basierend auf dem Messsignal zu ermitteln, wobei die Auswerteeinheit (12) ausgebildet ist, den Alterungszustand des Elastomerprodukts (4) basierend oder außerdem basierend auf der Resonanzfrequenz (f2, f3, f4) zu ermitteln.

7. System (2) nach einem der vorhergehenden Ansprüche, **dadurch gekennzeichnet, dass** von der Auswerteeinheit (12) eine Referenzfrequenz gespeichert ist, wobei die Auswerteeinheit (12) ausgebildet ist, den Alterungszustand des Elastomerprodukts (4) außerdem basierend auf der Referenzfrequenz zu ermitteln.

8. System (2) nach dem vorhergehenden Anspruch, **dadurch gekennzeichnet, dass** das erste Elastomermaterial (6) elektrisch isolierend ausgebildet ist.

9. System (2) nach dem vorhergehenden Anspruch, **dadurch gekennzeichnet, dass** das zweite Elastomermaterial als ein zweites, elektrisch leitfähiges Gummimaterial ausgebildet ist.

10. System (2) nach einem der vorhergehenden Ansprüche, **dadurch gekennzeichnet, dass** der Elastomerresonator (8) rein passiv ausgebildet ist.

11. System (2) nach einem der vorhergehenden Ansprüche, **dadurch gekennzeichnet, dass** der Metallresonator (18) rein passiv ausgebildet ist.

12. System (2) nach einem der vorhergehenden Ansprüche, **gekennzeichnet durch** eine mit der Auswerteeinheit (12) gekoppelte Signalschnittstelle (22) zur Übertragung eines Statussignals, das den von der Auswerteeinheit (12) ermittelbaren Alterungszustand des Elastomerprodukts (4) repräsentiert.

13. System (2) nach einem der vorhergehenden Ansprüche, **dadurch gekennzeichnet, dass** das Elastomerprodukt (4) ein Reifen, eine Luftfeder, ein Gummilager, ein Förderband, ein Riemen oder ein Schlauch ist.

## Claims

1. System (2) for determining a state of health of an elastomer product (4), the system (2) comprising:
an elastomer product (4) containing a first elastomer material (6),
a transceiver (10), and
an evaluation unit (12) coupled to the transceiver (10),
wherein the elastomer product (4) comprises an elastomer resonator (8) and a metal resonator (18),
wherein the elastomer resonator (8) is embedded in the first elastomer material (6) of the elastomer product (4),
wherein the elastomer resonator (8) is formed by a strip of a second, electrically conductive elastomer material,
wherein the metal resonator (18) is arranged on an outer side (20) of the elastomer product (4) so that the metal resonator (18) and the elastomer resonator (8) are arranged opposite and free of contact from one another,
wherein the metal resonator (18), the elastomer resonator (8) and a portion (24) of the first elastomer material (6) that is arranged between the metal resonator (18) and the elastomer resonator (8) form a measurement resonator (26), the reflection coefficient (s2, s3, s4) of which is dependent on the permittivity of the first elastomer material (6) and on the electrical conductivity of the elastomer resonator (8),
wherein the transceiver (10) is designed to generate an alternating electromagnetic field (16),
wherein the measurement resonator (26) is designed to be coupled into the alternating field (16) and to modify the alternating field (16) in such a way that the alternating field (16) has a resonant frequency (f2, f3, f4) marked by the reflection coefficient (s2, s3, s4) of the measurement resonator (26),
wherein the transceiver (10) is designed to detect the alternating field (16) and to generate a measurement signal that represents the alternating field (16) and/or the resonant frequency (f2, f3, f4) of the alternating field (16), and
wherein the evaluation unit (12) is designed to determine a state of health of the elastomer product (4) on the basis of the measurement signal.

2. System (2) according to the preceding claim, **characterized in that** the elastomer resonator (8) is arranged at least substantially centrally in the first elastomer material (6).

3. System (2) according to either of the preceding claims, **characterized in that** the metal resonator (18) is in the form of a dipole metal resonator.

4. System (2) according to one of the preceding claims, **characterized in that** the elastomer resonator (8) is arranged at least substantially parallel to the metal resonator (18) at a predetermined distance D of between 1 mm and 10 mm.

5. System (2) according to one of the preceding claims, **characterized in that** the transceiver (10) is designed to generate an alternating electromagnetic field (16) having a central base frequency of between 80 MHz and 120 MHz.

6. System (2) according to one of the preceding claims, **characterized in that** the evaluation unit (12) is designed to determine the resonant frequency (f2, f3, f4) of the alternating field (16) on the basis of the measurement signal, the evaluation unit (12) being designed to determine the state of health of the elastomer product (4) on the basis or additionally on the basis of the resonant frequency (f2, f3, f4).

7. System (2) according to one of the preceding claims, **characterized in that** the evaluation unit (12) stores a reference frequency, the evaluation unit (12) being designed to determine the state of health of the elastomer product (4) additionally on the basis of the reference frequency.

8. System (2) according to the preceding claim, **characterized in that** the first elastomer material (6) is in electrically insulating form.

9. System (2) according to the preceding claim, **characterized in that** the second elastomer material is in the form of a second, electrically conductive rubber material.

10. System (2) according to one of the preceding claims, **characterized in that** the elastomer resonator (8) is in purely passive form.

11. System (2) according to one of the preceding claims, **characterized in that** the metal resonator (18) is in purely passive form.

12. System (2) according to one of the preceding claims, **characterized by** a signal interface (22) coupled to the evaluation unit (12) for the purpose of transmitting a status signal that represents the state of health of the elastomer product (4) determinable by the evaluation unit (12).

13. System (2) according to one of the preceding claims, **characterized in that** the elastomer product (4) is a tyre, an air spring, a rubber bearing, a conveyor belt, a belt or a hose.

## Revendications

1. Système (2) de détermination d'un état de vieillissement d'un produit élastomère (4), le système (2) comportant :
un produit élastomère (4) ayant un premier matériau élastomère (6),
un émetteur-récepteur (10), et
une unité d'évaluation (12) qui est couplée à l'émetteur-récepteur (10),
le produit élastomère (4) comportant un résonateur élastomère (8) et un résonateur métallique (18),
le résonateur élastomère (8) étant noyé dans le premier matériau élastomère (6) du produit élastomère (4),
le résonateur élastomère (8) étant formé par une bande d'un deuxième matériau élastomère électriquement conducteur,
le résonateur métallique (18) étant disposé sur un côté extérieur (20) du produit élastomère (4) de sorte que le résonateur métallique (18) et le résonateur élastomère (8) soient disposés face à face et sans contact,
le résonateur métallique (18), le résonateur élastomère (8) et une partie (24) du premier matériau élastomère (6) qui est disposée entre le résonateur métallique (18) et le résonateur élastomère (8) forment un résonateur de mesure (26) dont le coefficient de réflexion (s2, s3, s4) dépend de la permittivité du premier matériau élastomère (6) et de la conductivité électrique du résonateur élastomère (8),
l'émetteur-récepteur (10) étant conçu pour générer un champ électromagnétique alternatif (16),
le résonateur de mesure (26) étant conçu pour insérer par couplage dans le champ alternatif (16) et le champ alternatif (16) étant modifié de manière à ce que le champ alternatif (16) présente une fréquence de résonance (f2, f3, f4) marquée par le coefficient de réflexion (s2, s3, s4) du résonateur de mesure (26),
l'émetteur-récepteur (10) étant conçu pour détecter le champ alternatif (16) et pour générer un signal de mesure qui représente le champ alternatif (16) et/ou la fréquence de résonance (f2, f3, f4) du champ alternatif (16), et
l'unité d'évaluation (12) étant conçue pour déterminer un état de vieillissement du produit élastomère (4) sur la base du signal de mesure.

2. Système (2) selon la revendication précédente, **caractérisé en ce que** le résonateur élastomère (8) est disposé au moins sensiblement au milieu dans le premier matériau blastomère (6).

3. Système (2) selon l'une des revendications précédentes, **caractérisé en ce que** le résonateur métallique (18) est conçu sous la forme d'un résonateur métallique dipôle.

4. Système (2) selon l'une des revendications précédentes, **caractérisé en ce que** le résonateur élastomère (8) est disposé au moins sensiblement parallèlement au résonateur métallique (18) à une distance prédéterminée D comprise entre 1 mm et 10 mm.

5. Système (2) selon l'une des revendications précédentes, **caractérisé en ce que** l'émetteur-récepteur (10) est conçu pour générer un champ électromagnétique alternatif (16) ayant une fréquence de base moyenne comprise entre 80 MHz et 120 MHz.

6. Système (2) selon l'une des revendications précédentes, **caractérisé en ce que** l'unité d'évaluation (12) est conçue pour déterminer la fréquence de résonance (f2, f3, f4) du champ alternatif (16) sur la base du signal de mesure, l'unité d'évaluation (12) étant conçue pour déterminer l'état de vieillissement du produit élastomère (4) sur la base ou en plus sur la base de la fréquence de résonance (£2, f3, f4).

7. Système (2) selon l'une des revendications précédentes, **caractérisé en ce qu'**une fréquence de référence est mémorisée par l'unité d'évaluation (12), l'unité d'évaluation (12) étant conçue pour déterminer l'état de vieillissement du produit élastomère (4) en plus sur la base de la fréquence de référence.

8. Système (2) selon la revendication précédente, **caractérisé en ce que** le premier matériau élastomère (6) est conçu pour être électriquement isolant.

9. Système (2) selon la revendication précédente, **caractérisé en ce que** le deuxième matériau élastomère est conçu sous la forme d'un deuxième matériau caoutchouteux électriquement conducteur.

10. Système (2) selon l'une des revendications précédentes, **caractérisé en ce que** le résonateur élastomère (8) est conçu pour être purement passif.

11. Système (2) selon l'une des revendications précédentes, **caractérisé en ce que** le résonateur métallique (18) est conçu pour être purement passif.

12. Système (2) selon l'une des revendications précédentes, **caractérisé par** une interface de signal (22) couplée à l'unité d'évaluation (12) et destinée à transmettre un signal d'état qui représente l'état de vieillissement du produit élastomère (4) qui peut être déterminé par l'unité d'évaluation (12).

13. Système (2) selon l'une des revendications précédentes, **caractérisé en ce que** le produit élastomère (4) est un pneumatique, un amortisseur pneumatique, un support en caoutchouc, une bande transporteuse, une courroie ou un tuyau.
